# EUROPÄISCHE PATENTANMELDUNG

(11) **EP 4 825 348 A1**
(43) Veröffentlichungstag der Anmeldung: **21.10.2026**
(21) Anmeldenummer: 25171076.0
(22) Anmeldetag: 16.04.2025
(51) Int. Cl.: A61F 2/52

(54) **KONKAVE BRUSTPROTHESE MIT WULST**

(71) Anmelder: Edelweiss Basics GmbH & Co. KG, 83098 Brannenburg (DE)
(72) Erfinder: WEBER-UNGER, Georg, 6330 Kufstein (AT)
(74) Vertreter: Kador & Partner Part mbB

(57) **Zusammenfassung**

Gegenstand der Erfindung ist eine Brustprothese (10) umfassend einen schalenförmigen Körper (1) der eine konvexe Vorderseite sowie eine an die Vorderseite anschließende konkave Rückseite (2) aufweist, wobei auf der konkaven Rückseite (2) am Unterbrustrand (8) der Brustprothese (10) ein Wulst (3) ausgebildet ist, der an eine Innenfläche (7) des schalenförmigen Körpers (1) angrenzt und an einem Übergang zu der Innenfläche (7) eine Hinterschneidung (7a) aufweist.

## Beschreibung

Die Erfindung betrifft eine konkave Brustprothese zum Tragen in einer linken oder rechten Büstenschale eines Büstenhalters oder Korseletts nach einer Brustamputation bei Frauen, insbesondere eine konkave Brustprothese mit Wulst.

Konkave Brustprothesen sind beispielsweise aus EP 3 549 554 A1 bekannt. Diese offenbart eine symmetrische Brustprothese mit konkaven Seitenrändern und einer spezifischen Breite zu Höhe Verhältnis der Rückseite der Brustprothese.

Der Erfindung liegt die Aufgabe zugrunde, eine Brustprothese bereitzustellen, die in Bezug auf Schwingungsverhalten, Stabilität und Tragekomfort gegenüber der in EP 3 549 554 A1 gezeigten Brustprothese verbessert ist.

Zur Lösung der Aufgabe werden die Merkmale des unabhängigen Anspruchs 1 vorgeschlagen. Vorteilhafte Ausgestaltungen finden sich in den abhängigen Ansprüchen.

Gegenstand der Erfindung ist eine Brustprothese umfassend einen schalenförmigen Körper, der eine konvexe Vorderseite sowie eine an die Vorderseite anschließende konkave Rückseite aufweist.

Der schalenförmige Körper ist vorzugsweise einstückig ausgebildet, insbesondere gegossen oder aus einem Materialblock geschnitten, und aus einem Elastomer oder einem Thermoplast gefertigt. Bei dem Elastomer kann es sich insbesondere um Silikon handeln und als bevorzugte Thermoplaste können ein thermoplastisches Elastomer wie thermoplastisches Polyurethan oder thermoplastisches Gummi verwendet werden. Der schalenförmige Körper kann insbesondere aus einem Elastomer- oder Thermoplastschaum im Spritzgussverfahren gefertigt sein. Dabei kann der Elastomer- oder Thermoplastschaum ein chemisches Treibmittel oder ein physikalisches Treibmittel, insbesondere Mikrosphären enthalten. Es ist ebenso möglich, dass der schalenförmige Körper aus einem geschäumten Elastomer- oder Thermoplastblock geschnitten ist.

Bei der erfindungsgemäßen Brustprothese kann es sich insbesondere um eine symmetrische Brustprothese handelt, deren Symmetrieachse mittig und vertikal durch die Brustprothese verläuft.

Die konvexe Vorderseite des schalenförmigen Körpers bildet insbesondere eine Form einer weiblichen Brust nach, während die konkave Rückseite, die einem Körper einer Trägerin zugewandt ist, eine Aushöhlung aufweist und dazu eingerichtet ist, nur auf ihren Rändern am Körper der Trägerin aufzuliegen, wodurch die Brustprothese eine gute Belüftung von darunter liegender Haut und Platz für verbliebenes Brustgewebe bietet.

Auf der konkaven Rückseite des schalenförmigen Körpers ist am Unterbrustrand der Brustprothese ein Wulst ausgebildet, der an eine Innenfläche des schalenförmigen Körpers angrenzt und an einem Übergang zu der Innenfläche eine Hinterschneidung aufweist. Der Wulst kann am Unterbrustrand insbesondere den Anschluss der konkaven Rückseite des schalenförmigen Körpers an die konvexe Vorderseite ausbilden. Insbesondere kann in einer Querschnittsansicht des Wulstes dessen Oberfläche im Bereich zwischen dem Unterbrustrand und der Innenfläche des schalenförmigen Körpers zumindest teilweise eine Kreisbogenform aufweisen, wobei die Wulstoberfläche zu der Innenfläche hin abfällt.

Der Unterbrustrand bildet üblicherweise einen unteren Rand der Brustprothese aus, der am Brustkorb der Trägerin anliegt und sich nach außen bzw. oben erstreckt, insbesondere symmetrisch. Dabei entspricht vorzugsweise zumindest ein Teil des Verlaufs des Unterbrustrandes einem Kreisbogen.

Der Wulst führt zu einer Materialansammlung am Unterbrustrand der Brustprothese, die den Schwerpunkt derselben nach unten verlagert. Auf diese Weise kann ein Schwingverhalten der Brustprothese an das einer natürlichen Brust angepasst werden, insbesondere in Verbindung mit der Hinterschneidung am Übergang zwischen dem Wulst und der Innenfläche des schalenförmigen Körpers. Zudem kann mittels des Wulstes ein unterer Brustbereich der Prothese stabilisiert und deren Tragekomfort erhöht werden.

Gemäß einer Ausführungsform kann eine Dicke des Wulstes in Umfangsrichtung des schalenförmigen Körpers in dessen Mitte am größten sein und nach außen hin abnehmen. Dabei kann eine Breite des Wulstes über seine gesamte Länge konstant sein oder variieren. Beispielsweise kann der Wulst an seinen Enden breiter ausgeführt sein. Unter der Dicke des Wulstes soll dessen Ausdehnung in vertikaler Richtung, unter der Breite des Wulstes dessen Ausdehnung in horizontaler Richtung und unter der Länge des Wulstes dessen tangentiale Ausdehnung, d.h. dessen Ausdehnung in Umfangsrichtung des schalenförmigen Körpers verstanden werden. Die maximale Dicke des Wulstes in der Mitte des Unterbrustrandes führt zu einer weiteren Schwerpunktverlagerung der Brustprothese nach unten, wodurch deren Schwingverhalten nochmals verbessert wird.

Gemäß einer Ausführungsform können auf der konkaven Rückseite des schalenförmigen Körpers an mindestens ein tangentiales Ende des Wulstes ein oder mehrere Stützränder angrenzen. Ein tangentiales Ende des Wulstes soll ein Wulstende in Umfangsrichtung des schalenförmigen Körpers bezeichnen. Insbesondere können sich an beide tangentiale Enden des Wulstes jeweils ein oder mehrere Stützränder anschließen. Zweckmäßigerweise kann der Wulst an seinen beiden tangentialen Enden kontinuierlich in die Stützränder übergehen.

In einer Ausführungsform können die Stützränder in horizontaler Richtung jeweils hintereinander angeordnet sein. Insbesondere können dabei ein erster und ein zweiter Stützrand beabstandet voneinander angeordnet sein. Beispielsweise kann der erste Stützrand entlang einer Außenkontur des schalenförmigen Körpers auf dessen Rückseite verlaufen, während der zweite Stützrand entlang einer Kontur der Innenfläche des schalenförmigen Körpers verlaufen kann. Die Begriffe "erster" und "zweiter" Stützrand dienen dabei lediglich zur Unterscheidung von zwei unterschiedlichen Stützrändern und begrenzen deren Anzahl nicht auf zwei.

Gemäß einer Ausführungsform kann der schalenförmige Körper in einer Vorderansicht eine dreiecksähnliche Form mit einen oberen Ausläufer und zwei seitlichen Ausläufern aufweisen, wobei sich der Unterbrustrand zwischen denen beiden seitlichen Ausläufern erstreckt. Mit oberem Ausläufer ist jener Ausläufer gemeint, der im Tragezustand der Brustprothese zur Schulter der Trägerin weist. Die seitlichen Ausläufer erstrecken sich in diesem Fall von der Symmetrieachse der Brustprothese nach links und rechts. Aufgrund der Symmetrie der Brustprothese können die beiden seitlichen Ausläufer einander spiegelbildlich in Form und Größe entsprechen.

Vorzugsweise können ein Außenrand des obere Ausläufers sowie Außenränder der seitlichen Ausläufer ebenfalls zumindest teilweise eine Kreisbogenform aufweisen. Die an die tangentialen Enden des Wulstes anschließenden Stützränder können sich über die seitlichen Ausläufer erstrecken, um diese zu stabilisieren. Die Stützränder können dabei insbesondere in die seitlichen Ausläufer integriert z.B. in diese eingegossen sein.

Insbesondere können sich die Stützränder in Umfangsrichtung des schalenförmigen Körpers jeweils zwischen dem entsprechenden Ende des Wulstes und einer maximalen Ausdehnung der seitlichen Ausläufer erstrecken. Dadurch kann deren Stabilisierung im Hinblick auf das Schwingungsverhalten der Brustprothese optimiert werden.

Ein Bereich der Innenfläche des schalenförmigen Körpers, der an den Wulst am Unterbrustrand bzw. dessen Hinterschneidung angrenzt, kann dicker ausgeführt sein als ein Bereich der Innenfläche, der den oberen Ausläufer des schalenförmigen Körpers angrenzt bzw. in diesen übergeht. Dies trägt zusätzlich zur Stabilisierung des unteren Brustbereichs der Prothese bei und verbessert gleichzeitig deren Schwingverhalten.

Alternativ oder zusätzlich können auf der konkaven Rückseite des schalenförmigen Körpers eine oder mehrere Stützstreben angeordnet sein, die an den Wulst angrenzen und sich in vertikaler Richtung oder sternförmig über die Innenfläche des schalenförmigen Körpers erstrecken. Die Stützstreben können insbesondere in die Innenfläche der konkaven Rückseite integriert, z.B. in diese eingegossen sein.

Die Stützstreben können dabei insbesondere die Stabilität des schalenförmigen Körpers gegenüber einer Druckkraft auf dessen Vorderseite erhöhen und somit einem "Plattdrücken" der Brustprothese entgegenwirken. Da die Stützstreben nur wenig Material benötigen, tritt nahezu keine Gewichtserhöhung auf. Zudem erlaubt die konkave Form der Rückseite eine ausreichende Belüftung von unter der Prothese befindlicher Haut der Trägerin, wodurch der Tragekomfort gesteigert werden kann. Mit anderen Worten stellt die Erfindung eine Brustprothese mit erhöhter Stabilität bereit und verbesserter Belüftung bereit, die keine nennenswerte Gewichtszunahme zur Folge hat und eine unerwünschte Verformung der Prothese z.B. in einem Büstenhalter zuverlässig vermeiden kann.

Weitere Einzelheiten, Merkmale und Vorteile des Gegenstands der Erfindung ergeben sich aus der nachfolgenden Beschreibung der zugehörigen Figuren, in welchen beispielhaft bevorzugte Ausführungsformen der Erfindung dargestellt ist.

Darin zeigen in vereinfachter schematischer Darstellung
Figur 1 eine Rückansicht eines ersten Ausführungsbeispiels der erfindungsgemäßen Brustprothese,
Figur 2 eine Rückansicht eines zweiten Ausführungsbeispiels der erfindungsgemäßen Brustprothese,
Figur 3a eine Rückansicht eines dritten Ausführungsbeispiels der erfindungsgemäßen Brustprothese, und
Figur 3b eine Rückansicht eines vierten Ausführungsbeispiels der erfindungsgemäßen Brustprothese.

In den Figuren sind gleiche oder ähnliche Elemente mit gleichen Bezugszeichen versehen, so dass gegebenenfalls auf eine wiederholende Beschreibung verzichtet wird.

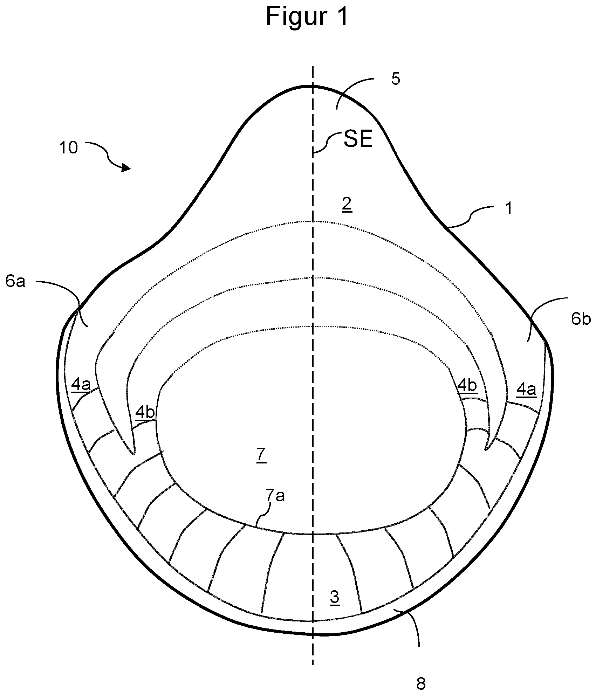

Die in Figur 1 in einer Rückansicht gezeigte Brustprothese 10 umfasst einen schalenförmigen Körper 1 mit einer konvexe Vorderseite (nicht dargestellt) und einer konkaven Rückseite 2. Der schalenförmige Körper 1 kann in diesem Ausführungsbeispiel insbesondere aus einem elastischen Thermoplast, z.B. aus thermoplastischem Polyurethan oder thermoplastischem Gummi gefertigt sein. Es ist jedoch ebenso möglich, dass der schalenförmige Körper 1 aus einem Elastomer z.B. Silikon gefertigt ist. Der schalenförmige Körper kann insbesondere aus einem Elastomer- oder Thermoplastschaum im Spritzgussverfahren gefertigt sein. Dabei kann der Elastomer- oder Thermoplastschaum ein chemisches Treibmittel oder ein physikalisches Treibmittel, insbesondere Mikrosphären enthalten. Alternativ kann der schalenförmige Körper aus einem geschäumten Elastomer- oder Thermoplastblock geschnitten sein.

Der schalenförmiger Körper 1 weist vorliegend eine dreiecksähnliche Form mit einer Symmetrieebene SE sowie einem oberen Ausläufer 5 und zwei seitlichen Ausläufern 6a, 6b auf, zwischen denen sich ein Unterbrustrand 8 der Brustprothese 10 erstreckt. Der obere Ausläufer 5, die seitlichen Ausläufer 6a, 6b und der Unterbrustrand 8 haben zumindest teilweise einen bogenförmigen Verlauf.

Auf der konkaven Rückseite 2 des schalenförmigen Körpers 1 ist am Unterbrustrand 8 ein Wulst 3 angeordnet, der an eine Innenfläche 7 des schalenförmigen Körpers 1 angrenzt und an einem Übergang zu der Innenfläche 7 eine Hinterschneidung 7a aufweist. Der Wulst 3 weist an der Symmetrieachse SE, d.h. in der Mitte des Unterbrustrandes 8 seine größte Dicke auf und wird zu seinen tangentialen Enden hin kontinuierlich dünner.

Der Wulst 3 führt zu einer Materialansammlung am Unterbrustrand 8 der Brustprothese 10, die den Schwerpunkt derselben nach unten verlagert. Auf diese Weise kann ein Schwingverhalten der Brustprothese 10 an das einer natürlichen Brust angepasst werden, insbesondere in Verbindung mit der Hinterschneidung 7a am Übergang zwischen dem Wulst 3 und der Innenfläche 7 des schalenförmigen Körpers 1. Zudem kann mittels des Wulstes 3 ein unterer Brustbereich der Prothese 10 stabilisiert und deren Tragekomfort erhöht werden.

In Umfangsrichtung des schalenförmigen Körpers 1 grenzen zwei Stützränder 4a, 4b jeweils an ein Ende des Wulstes 3 an, die in horizontaler Richtung hintereinander und beabstandet zueinander angeordnet sind. Insbesondere schließen sich die beiden Stützränder 4a, 4b direkt an den Wulst 3 an bzw. dieser geht an seinen Enden in die Stützränder 4a, 4b über. Dabei verläuft ein äußerer Stützrand 4a parallel zu einer Außenkontur des schalenförmigen Körpers 1 und ein innerer Stützrand 4b parallel zu einer Kontur der Innenfläche 7 des schalenförmigen Körpers 1.

Vorliegend erstrecken sich die Stützränder 4a, 4b in Umfangsrichtung des schalenförmigen Körpers1 im Wesentlichen von den Wulstenden und bis zu einer maximalen Ausdehnung der seitlichen Ausläufer 6a, 6b. Mit anderen Worten erstrecken sich die Stützränder 4a, 4b von den Wulstenden am Unterbrustrand 8 bis zu einem Bereich, in dem die Brustprothese 10 ihre maximale Breite aufweist. Dies ermöglicht es die Stabilität der seitlichen Ausläufer 6a, 6b zu erhöhen und gleichzeitig das Schwingverhalten einer natürlichen Brust gut nachzubilden. Die Stützränder 4a, 4b können dabei insbesondere in die seitlichen Ausläufer 6a, 6b integriert z.B. in diese eingegossen sein.

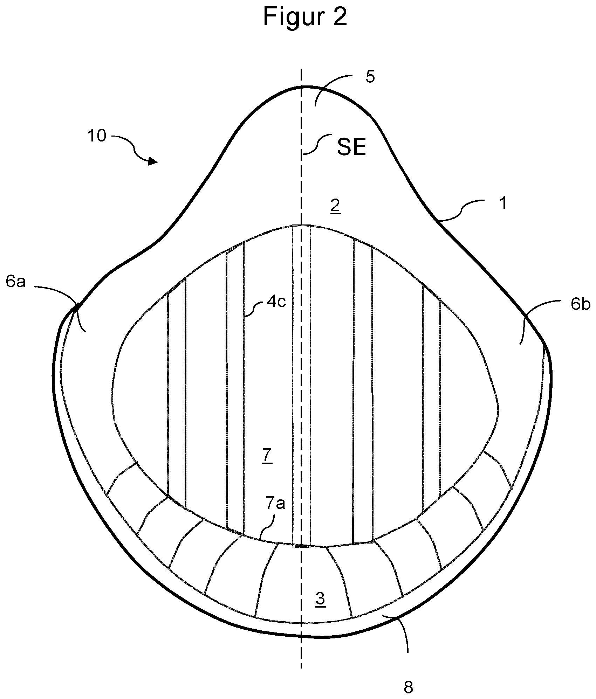

Die in Figur 2 gezeigte Brustprothese gemäß einem zweiten Ausführungsbeispiel der Erfindung umfasst ebenfalls einen schalenförmigen Körper 1 aus einem elastischen Thermoplast mit einer konvexen Vorderseite und einer konkave Rückseite 2, der eine symmetrische dreiecksähnliche Form mit einem oberen Ausläufer 5 und zwei seitlichen Ausläufern 6a, 6b aufweist, zwischen denen sich ein Unterbrustrand 8 der Brustprothese 10 erstreckt.

Wie im ersten Ausführungsbeispiel ist auch hier am Unterbrustrand 8 ein Wulst 3 auf der Rückseite 2 des schalenförmigen Körpers 1 angeordnet, der an eine Innenfläche 7 des schalenförmigen Körpers 1 angrenzt und an einem Übergang zu der Innenfläche 7 eine Hinterschneidung 7a aufweist. Mit anderen Worten ist auch hier ein Wulst 3 zwischen dem Unterbrustrand 8 und der Innenfläche 7 des schalenförmigen Körpers ausgebildet.

Im Unterschied zu dem ersten Ausführungsbeispiel sind vorliegend jedoch auf der konkaven Rückseite 2 des schalenförmigen Körpers 1 mehrere Stützstreben 4c (vorliegend fünf Stützstreben 4c) angeordnet, die an den Wulst 3 angrenzen und sich in vertikaler Richtung über die Innenfläche 7 des schalenförmigen Körpers 1 erstrecken. Die Stützstreben 4c können insbesondere in die Innenfläche 7 der konkaven Rückseite 2 integriert, z.B. in diese eingegossen sein.

Die Stützstreben 4c können dabei insbesondere die Stabilität des schalenförmigen Körpers 1 gegenüber einer Druckkraft auf dessen Vorderseite erhöhen und somit einem "Plattdrücken" der Brustprothese 10 entgegenwirken. Da die Stützstreben 4c nur wenig Material benötigen, tritt nahezu keine Gewichtserhöhung auf.

Die in Figur 2 gezeigte Brustprothese 10 weist auf ihrer konkaven Rückseite 2 keine an die Enden des Wulstes 3 angrenzenden Stützränder 4a, 4b auf, sondern die Stabilität des schalenförmigen Körpers 1 wird allein durch die vertikalen Streben 4c auf der Innenfläche 7 bewirkt. Es ist jedoch ebenso möglich, zusätzlich zu den vertikalen Streben 4c einen oder mehrere Stützränder 4a, 4b wie in Figur 1 gezeigt auf der Rückseite 2 des schalenförmigen Körpers 1 anzubringen.

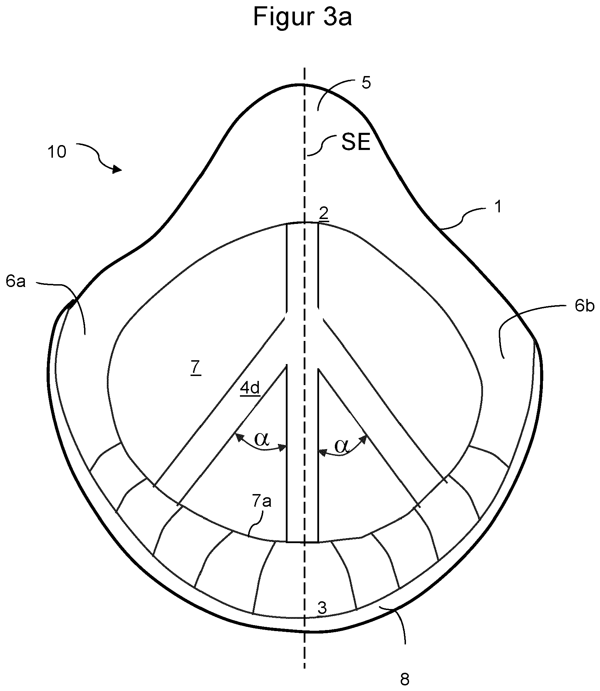

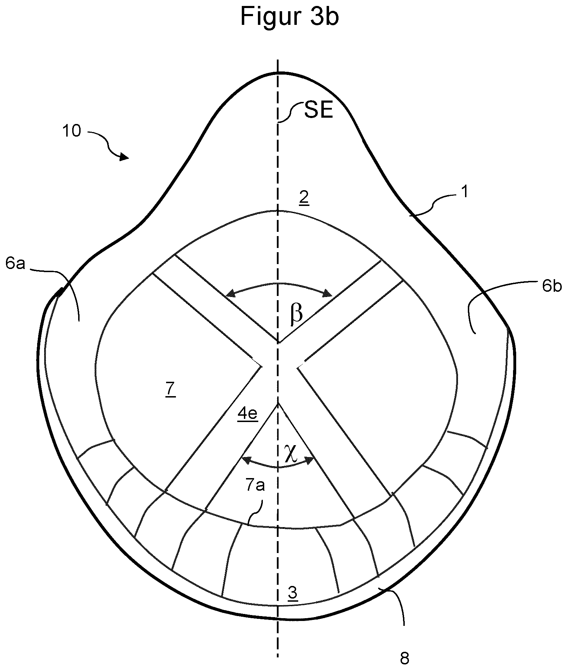

In den Figuren 3a und 3b sind weitere Ausführungsbeispiele der Erfindung gezeigt, die sich von dem in Figur 2 dargestellten Ausführungsbeispiel lediglich dadurch unterscheiden, dass die Stützstreben 4d, 4e auf der konkaven Rückseite 2 des schalenförmigen Körpers 1 nicht vertikal, sondern sternförmig über dessen Innenfläche 7 verlaufen.

Dabei weist die in Figur 3a gezeigte Brustprothese 10 eine mittlere Strebe 4d auf, die sich angrenzend an den Wulst 3 in vertikaler Richtung entlang der Symmetrieachse SE über die Innenfläche 7 erstreckt. Zusätzlich sind zwei um einen Winkel α zu der vertikalen Strebe 4d geneigte Streben 4d vorhanden, die sich von einem tiefsten Punkt des schalenförmigen Körpers 1 bis zu dem Wulst erstrecken.

Die in Figur 3b gezeigte Brustprothese 10 weist vier Streben 4e auf, die sich jeweils von dem tiefsten Punkt des schalenförmigen Körpers 1 bis zu der Kontur der Innenfläche 7 erstrecken. Dabei sind zwei obere Streben 4e in einem Winkel β und zwei untere Streben in einem Winkel χ zueinander angeordnet, wobei der Winkel β größer als der Winkel χ ist. Dadurch erstrecken sich die sternförmig angeordneten Streben über die gesamte Innenfläche 7 auf der konkaven Rückseite 2 des schalenförmigen Körpers.

Diese führt zu einer Versteifung des schalenförmigen Körpers 1, wodurch eine unerwünschte Verformung, insbesondere eine Abflachung der elastischen Brustprothese bei Druckeinwirkung, z.B. in einem Büstenhalter vermieden werden kann.

Die in den Figuren 3a und 3b gezeigten Brustprothesen 10 weisen auf ihrer konkaven Rückseite 2 keine an die Enden des Wulstes 3 angrenzenden Stützränder 4a, 4b auf, sondern die Stabilität des schalenförmigen Körpers 1 wird allein durch die sternförmig angeordneten Streben 4d, 4e auf der Innenfläche 7 bewirkt. Es ist jedoch ebenso möglich, zusätzlich zu den sternförmig angeordneten Streben 4d, 4e einen oder mehrere Stützränder 4a, 4b wie in Figur 1 gezeigt auf der Rückseite 2 des schalenförmigen Körpers 1 anzubringen.

Anhand der gezeigten Ausführungsbeispiele der Erfindung wird deutlich, dass die gezeigte Brustprothese 10 über eine hohe Stabilität verfügt und gleichzeitig das Schwingungsverhalten einer natürlichen Brust abbildet. Dies trägt beides zu einem erhöhten Tragekomfort der Brustprothese 10 bei.

### Bezugszeichenliste

- 1: Schalenförmiger Körper der der Brustprothese (10)
- 2: Konkave Rückseite des schalenförmigen Körpers (1)
- 3: Wulst
- 4a, b: Stützränder
- 4c, d, e: Stützstreben
- 5: Oberer Ausläufer
- 6a: Linker seitlicher Ausläufer
- 6b: Rechter seitlicher Ausläufer
- 7: Innenfläche auf der konkaven Rückseite (2)
- 8: Unterbrustrand
- 10: Brustprothese
- SE: Symmetrieachse

## Patentansprüche

1. Brustprothese (10) umfassend einen schalenförmigen Körper (1) der eine konvexe Vorderseite sowie eine an die Vorderseite anschließende konkave Rückseite (2) aufweist, wobei auf der konkaven Rückseite (2) am Unterbrustrand (8) der Brustprothese (10) ein Wulst (3) ausgebildet ist, der an eine Innenfläche (7) des schalenförmigen Körpers (1) angrenzt und an einem Übergang zu der Innenfläche (7) eine Hinterschneidung (7a) aufweist.

2. Brustprothese (10) nach Anspruch 1, wobei eine Dicke des Wulstes (3) in Umfangsrichtung des schalenförmigen Körpers (1) in der Mitte des Unterbrustrandes (8) am größten ist und nach außen hin abnimmt.

3. Brustprothese (10) nach Anspruch 1 oder 2, wobei auf der konkaven Rückseite (2) des schalenförmigen Körpers (1) in Umfangsrichtung des schalenförmigen Körpers (1) an mindestens ein Ende des Wulstes (3) ein oder mehrere Stützränder (4a, 4b) angrenzen.

4. Brustprothese (10) nach Anspruch 3, wobei die Stützränder (4a, 4b) in horizontaler Richtung hintereinander angeordnet sind

5. Brustprothese (10) nach Anspruch 4, wobei ein erster Stützrand (4a, 4b) und ein zweiter Stützrand (4b) beabstandet voneinander angeordnet sind.

6. Brustprothese (10) nach einem der vorstehenden Ansprüche, wobei der schalenförmige Körper (1) in einer Vorderansicht eine dreiecksähnliche Form mit einem oberen Ausläufer (5) und zwei seitlichen Ausläufern (6a, 6b) aufweist, und sich der Unterbrustrand (8) zwischen denen beiden seitlichen Ausläufern (6a, 6b) erstreckt.

7. Brustprothese (10) nach Anspruch 5, wobei sich die Stützränder (4a, 4b) in Umfangsrichtung des schalenförmigen Körpers (1) zwischen dem mindestens einem Ende des Wulstes (3) und einer maximalen Ausdehnung der seitlichen Ausläufer (6a, 6b) erstrecken.

8. Brustprothese (10) nach einem der vorstehenden Ansprüche, wobei auf der konkaven Rückseite (2) des schalenförmigen Körpers (1) eine oder mehrere Stützstreben (4c, 4d, 4e) angeordnet sind, die an den Wulst (3) angrenzen und sich in vertikaler Richtung oder sternförmig über die Innenfläche (7) des schalenförmigen Körpers (1) erstrecken.

9. Brustprothese (10) nach einem der vorstehenden Ansprüche, wobei der schalenförmige Körper (1) einstückig ausgebildet, insbesondere gegossen oder aus einem Materialblock geschnitten, ist.

10. Brustprothese (10) nach einem der vorstehenden Ansprüche, wobei der schalenförmige Körper (1) aus einem Elastomer, insbesondere aus Silikon oder aus einem Thermoplast, insbesondere aus einem thermoplastischen Elastomer, wie thermoplastischem Polyurethan oder thermoplastischem Gummi, gefertigt ist.

11. Brustprothese (10) nach einem der vorstehenden Ansprüche, wobei der schalenförmige Körper (1) aus einem Elastomer- oder Thermoplastschaum im Spritzgussverfahren gefertigt ist.

12. Brustprothese (10) nach Anspruch 11, wobei der Elastomer- oder Thermoplastschaum ein chemisches Treibmittel oder ein physikalisches Treibmittel, insbesondere Mikrosphären enthält.

13. Verwendung von einem Elastomer, insbesondere Silikon oder einem Thermoplast, insbesondere einem thermoplastischen Elastomer wie thermoplastisches Polyurethan oder thermoplastisches Gummi als Material für eine Brustprothese (10).
